# EUROPEAN PATENT SPECIFICATION

(11) **EP 1 330 778 B1**
(45) Date of publication and mention of the grant of the patent: **28.04.2004**
(21) Application number: 01992981.9
(22) Date of filing: 30.10.2001
(51) Int. Cl.: G06K 11/08, G06F 3/033

(54) **METHOD AND APPARATUS FOR MONITORING A TARGET**
VERFAHREN UND VORRICHTUNG ZUR ÜBERWACHUNG EINES ZIELS
PROCEDE ET APPAREIL PERMETTANT DE CONTROLER UNE CIBLE

(30) Priority: 31.10.2000 GB 0027143
(43) Date of publication of application: 30.07.2003
(73) Proprietor: Malvern Scientific Solutions Limited, Suckley Worcestershire WR6 5DR (GB)
(72) Inventor: BEALE, Marc, Ivor, John, Suckley Worcestershire WR6 5DR (GB)
(74) Representative: Jackson, Derek Charles
(86) International application number: PCT/GB2001/004797
(87) International publication number: WO 2002/037412

(56) References cited:
- EP-A- 0 055 338
- US-A- 5 818 954

## Description

The present invention relates to a method and apparatus for monitoring a target, such as the eyes of a subject. More particularly, but not exclusively, the invention relates to a method and apparatus for monitoring a target, such as the eyes of a subject, as by tracking movement of the target.

The invention may be used, for example, to control an image on a display, such as a screen. Thus, the invention may be used to control the display of a part of a larger image and to pan and/or tilt the image on the display, changing the image displayed as required by tracking movement of the target, and/or to control the position and/or function of an image in the form of a cursor or the like on a computer screen according to movement of the target and/or to control a machine that may not incorporate a display (for example, by the user nodding his head to actuate a switch).

EP-A-0 055 338, on which the preambles of claims 1 and 12 are based, describes a means for eye-controlled user-machine communication in which information is transferred interactively between a user and an information processor by establishing the direction of the user's gaze or visual focus with relation to portions of a viewing surface. Thereafter the direction of the user's gaze or visual focus to a specific portion of the viewing surface conveys information. The direction of a user's gaze is established through a reflected signal from the operator's eyeball.

It is often desirable to show only part of a larger image on a display. The larger image may comprise, for example, a scene, a spreadsheet or a form that cannot, or cannot readily, be displayed as a whole. A mouse, trackball or keyboard can be used to pan and/or tilt the part of the overall image that is displayed, but these control means require the use of one or both of the user's hands and are therefore inconvenient. Moreover, these control means may already be in use for other functions and an alternative control means would be useful. It is desirable to be able to control the pan and/or tilt of such an image without the need for manual intervention or in addition to conventional manual control.

Conventional pattern recognition techniques which can be used in optical tracking require the processing of substantial amounts of data in real time. For example, an image of 500,000 pixels at 16 bits resolution (1Mbyte of data) is generally required to be processed at frame rate, some 25 times per second. Conventional techniques build up a two dimensional image as a pixel array which is held in memory and which is manipulated by a processor. These techniques require significant amounts of memory and processing power (both in terms of computational power and electrical power) with attendant high cost, together with sophisticated image processing software. Processing time can also be undesirably long.

Further, it is known to monitor a subject's eyes to measure "perclos" or for gaze tracking. Perclos, generally, is a measure of the proportion of time that a subject's eyes are closed, either completely or beyond a predetermined point. For example, perclos may be the measure of the proportion of time that a subject's eyes are between 80 percent and 100 percent closed. Often, the measuring of perclos must be done manually, such as by videotaping the subject, reviewing the tape, and measuring the subject's perclos. Such a method, of course, is not practical for many applications.

Another method of determining perclos involves the use of an image sensor, such as a video camera, and image processing software to monitor the subject, determine the location of the subject's eyes, and determine the subject's perclos. Such a method is, however, time consuming, costly and often cannot be performed in real time, thereby inhibiting if from being used to determine the drowsiness of a driver of a motor vehicle.

It is often difficult to locate and monitor the subject's eyes. For example, prior art apparatus often cannot distinguish between the subject's eyes and other sources of inhibiting if from being used to determine the drowsiness of a driver of a motor vehicle.

It is often difficult to locate and monitor the subject's eyes. For example, prior art apparatus often cannot distinguish between the subject's eyes and other sources of light and reflected light, such as is caused by dashboard lights, lights from other vehicles, and street lights. These problems are increased when the subject is wearing spectacles.

It is therefore an object of the present invention to provide a method and apparatus for monitoring a target which is easy to implement and relatively economical to manufacture.

According to one aspect of the present invention there is provided a method for monitoring at least one target, comprising the steps of providing a signal indicative of an image received by an image sensor, and processing the image signal to determine an amplitude of the image signal and to identify any part or parts of the signal having an amplitude greater than a predetermined threshold, and to identify location coordinates of the part or parts of the signal having an amplitude greater than the predetermined threshold, wherein the image signal is processed to determine the duration for which any part or parts of the image signal has an amplitude greater than the predetermined threshold and to select only the part or parts having a linear extent greater than a first predetermined value and/or less than a second predetermined value, the second predetermined value being longer than the first predetermined value.

According to another aspect of the present invention there is provided an apparatus for monitoring at least one target, comprising an image sensor for producing a signal indicative of an image received by the sensor, and means for processing the image signal, the processing means including comparator means for determining an amplitude of the image signal and for identifying any part or parts of the signal having an amplitude greater than a predetermined threshold, and including means for identifying location coordinates of the part or parts of the signal having an amplitude greater than the predetermined threshold, wherein the processing means includes means for determining the duration for which any part or parts of the image signal has an amplitude greater than the predetermined threshold and for selecting only the part or parts having a linear extent greater than a first predetermined value and/or less than a second predetermined value, the second predetermined value being longer than the first predetermined value.

Thus the monitoring apparatus according to the present invention is capable of processing the image signal in real time, as the signal is received, and there is no need to store the image for subsequent processing.

The invention may be used to control an image on a display, such as a screen. For example, the image displayed may be panned and/or tilted depending on the location of the radiation emitting device within the field of view.

The first predetermined value may be about 0.5 percent of the field of view of the imaging device. The second predetermined value may be about 5 percent of the field of view of the imaging device.

The processing means may include means for determining the distance of the target from the image sensor on the basis of the linear extent of the part or parts of the image signal having an amplitude greater than the predetermined threshold. Thus, the invention may be used to zoom in or out of an image depending on the distance between the imaging device and the target.

The radiation source may be selected from a visible emitter, a UV emitter and an infra-red emitter.

The image sensor may incorporate shutter means and means may be provided for pulsing the radiation source in synchronism with opening of the shutter.

Two sources of electromagnetic radiation may be provided, the sources having first and second wavelengths, the second wavelength being different to the first wavelength. The first wavelength may be in the range from 700 to 750 nm, preferably about 740 nm, and the second wavelength may be in the range from 800 to 1000 nm, preferably about 810 nm. Alternatively, the first wavelength may be about 880 nm and the second wavelength may be about 940 nm.

One of the first and second radiation sources may be substantially co-located with the image sensor and the other of the first and second radiation sources may be offset laterally relative to the image sensor.

A first polarising filter may be positioned in front of each of the first and second radiation sources and a second polarising filter, oriented to block polarised radiation from the first polarising filter, may be positioned in front of the image sensor. The polarising filters have the effect of eliminating specular reflections.

The processing means may include means for determining the amplitude of first and second spectral regions of the image signal, means for determining the ratio of the amplitudes of the first and second spectral regions, and means for selecting only the part or parts of the image signal having a ratio of at least, or not more than, a predetermined value. The first spectral region may represent the colour green or blue and the second spectral region may represent the colour red. The predetermined ratio value for the amplitude of the first spectral region to the amplitude of the second spectral region may be less than about 1.1.

As an alternative, the first and second radiation sources may be arranged in first and second planes parallel to the image sensor, the first and second planes being spaced in the direction of an optical axis of the image sensor. The first and second radiation sources may be substantially co-located with the image sensor.

Where the radiation sources are point sources, the intensity of radiation falls off in dependence upon the square of distance. Therefore the intensity of radiation reflected by the target is different for each of the first and second sources and the ratio of intensities can be used to determine the distance between the target and the image sensor. The coordinates of the target in space may be determined on the basis of the angular position of the target and the distance thereof from the image sensor.

For a better understanding of the present invention and to show more clearly how it may be carried into effect reference will now be made, by way of example, to the accompanying drawings in which:
Figure 1 is a diagrammatic illustration of one embodiment of an apparatus according to the present invention for monitoring a target;
Figure 2 illustrates diagrammatically one method for identifying the location of a target resulting from an optical tag in the monitoring apparatus according to the present invention;
Figure 3 illustrates diagrammatically another method for identifying the location of a target resulting from an optical tag in the monitoring apparatus of the present invention;
Figure 4 is a schematic circuit diagram for effecting the method illustrated in Figure 3;
Figure 5 is a diagrammatic illustration of another embodiment of an apparatus according to the present invention for monitoring a target;
Figure 6 is a circuit diagram illustrating a modification of the circuit diagram of Figure 4;
Figure 7 is a diagrammatic illustration of the display of part only of a larger image; and
Figures 8 and 9 illustrate diagrammatically an embodiment of the monitoring apparatus according to the present invention for identifying the location of a pointing device, such as a finger, in a 3-dimensional volume.

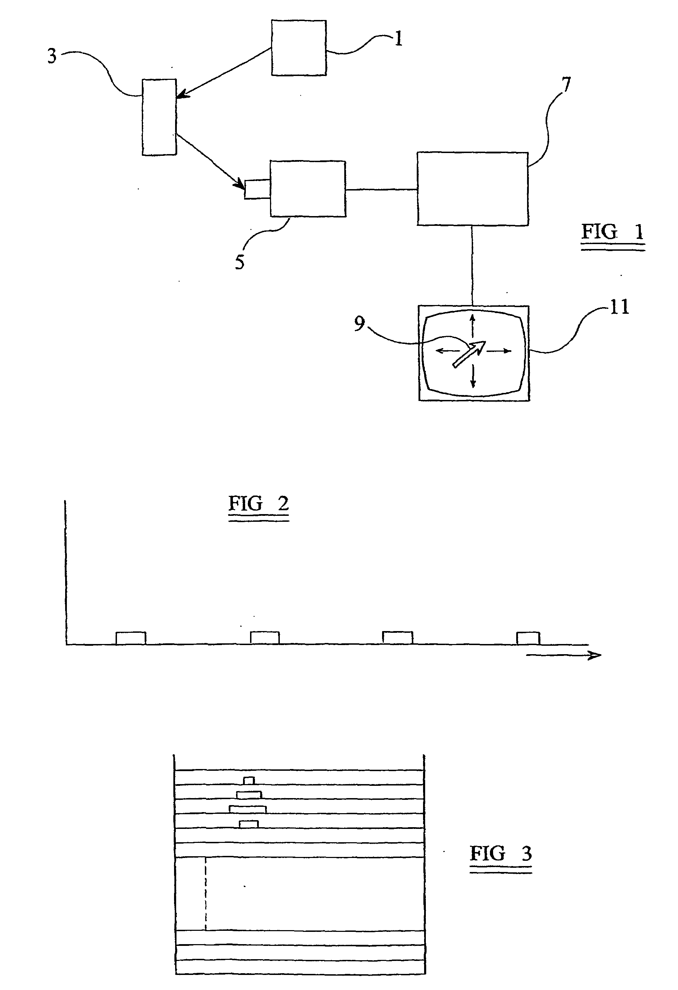

The monitoring apparatus shown diagrammatically in Figure 1 comprises a radiation source 1 which directs radiation towards at least one optical tag (target) in the form of a radiation emitting device 3, which radiation emitting device 3 emits radiation in response to incident radiation from the source 1. A single circular radiation emitting device allows orthogonal pan and tilt movements to be determined (and combinations thereof) together with range, while a single non-circular radiation emitting device or two radiation emitting devices permit, for example, relative positions to be determined, such as the elevation of one radiation emitting device relative to the other (i.e., tilt about an axis). The source 1 may be, for example, a source of visible, ultraviolet or, preferably, infra-red electromagnetic radiation and may comprise a filament lamp (with suitable filtration if necessary, for example to block out undesired visible radiation and/or to improve contrast), a light-emitting diode, ambient light or the like. The or each radiation emitting device 3 may comprise, for example, a retroreflective device, such as a layer of microbeads, corner-cubes or the human or an animal eye, which reflects incident radiation substantially towards the source thereof, or may comprise a luminescent or phosphorescent material which emits radiation in response to incident radiation. The or each radiation emitting device also comprises another feature (shape) which serves to distinguish emitted radiation from the remainder of the image scene. Further distinguishing features, such as a particular colour or pattern may also be provided. If desired, the source 1 may be incorporated into the emitting device 3.

Radiation from the or each radiation emitting device 3 is detected as a target by an imaging device 5, such as a colour or monochrome CCTV, digital imaging camera or other line scanning camera, which conventionally produces a serial (rasterised) image signal. If desired, to aid discrimination, radiation from the source 1 may be pulsed or a short shutter time (such as of the order of 100 microseconds) can be used to reduce the impact of ambient radiation on the image signal.

The or each radiation emitting device 3 is small relative to the field of vision of the imaging device 5 and, as will be explained in more detail hereinafter, is designed to produce a predetermined change in a region of the serial image signal which contrasts with the background scene. The location of the position of radiation emitted from the radiation emitting device 3 towards the imaging device 5 within the field of view of the imaging device is regarded as a target. The predetermined change in the signal is in practice a region of increased intensity represented by a higher signal level. The predetermined change in the signal permits the radiation from the radiation emitting device 3 to be selected from radiation from the background scene by simple means such as a voltage comparator which can be incorporated into a processor 7 or other suitable component, and the location of the target as represented by the predetermined change in the signal can be represented as cursor 9, for example, in a two dimensional display 11. The predetermined change in the signal can be recognised for example by identifying when the amplitude of the signal exceeds a predetermined threshold, and locating the rising (leading) and falling (trailing) edges of the signal where it exceeds the threshold, with the voltage comparator and filtering out those portions of the signal in which the time span between the rising and falling edges is too great or too small. The centre of the predetermined change in the signal can readily be identified, for example if desired for reasons of accuracy, as by determining the midpoint between the rising and falling edges.

A change in the angular position of the target (i.e., the position of radiation emitted from the radiation emitting device 3 towards the imaging device 5) relative to the imaging device within the field of view of the imaging device results in a change in the location of the predetermined change in the signal. The movement monitored can be used for any suitable purpose such as effecting corresponding movement of the cursor 9 on the display as illustrated or panning and/or tilting an image, or controlling an aspect of a computer game. Where the target is the subject's eyes, the presence or absence of a target and the size of that target can be used to determine perclos.

The location of the predetermined change in the signal can be identified within the field of view in a number of ways.

One method is illustrated in Figure 2, in which the serial image signal comprises a single field. That is to say, in effect the image has been reduced to a one dimensional array of sequential lines. In such a case the length of the signal is known and a simple timer incorporated into the processor 7 can be used to identify in real time the time lapse from the beginning of the signal to the location of the predetermined change (target) such as the leading edge of a region of increased signal strength. Multiple rising and falling edges can be used to build up multiple lines corresponding to the location of the region of predetermined change. A straightforward calculation can then give the x and y co-ordinates of the target which may then be displayed as a cursor or used for other purposes. The region of predetermined change can therefore be located without storing the entire image in memory and without sophisticated analysis of the stored image. On the contrary, it is possible to analyse the image in real time with a relatively inexpensive processor.

Another method is illustrated in Figure 3, in which the serial image signal comprises a predetermined number of lines each of predetermined period. In this case the image is treated as a two dimensional array. For example in the PAL system, each line scan period is 64 microseconds. The x and y co-ordinates can be determined in real time, the y co-ordinate by counting the line number and the x co-ordinate by identifying the time lapse from the beginning of the line to the location of the predetermined change (target) in the signal as described above. Once again, the predetermined change in the signal can be located without storing the entire image in memory and without sophisticated analysis of the stored image.

The predetermined change in the signal is identified by creating a substantial contrast between the region of change and the background scene in the form of a region having a particular geometrical shape. The predetermined change in the signal can further be identified by one or more of a region of higher intensity and a region which generates a predetermined sequence of signals such as stripes, or a region having a specific colour.

In one specific embodiment for implementing the embodiment of Figure 3 so as to detect a region of higher intensity, the imaging device 5 comprises a Philips monochrome camera VCM36 provided with an 8mm f4.0 lens, the camera being modified to set the automatic gain control at its minimum value and to set the shutter speed at 100 microseconds.

The radiation source 1 comprises eight Siemens SFH 487 880nm light emitting diodes arranged around the lens on as small a diameter as is practical. The diodes are driven in parallel supplying 8 amps for a period of 100 microseconds during each 20 millisecond field period, the illumination pulse being synchronised with the open period of the shutter of the camera. Alternatively, the illumination may be continuous, but it has been found that pulsed illumination can provide a gain of a factor of five or more in the suppression of ambient illumination.

For simplicity, pulses of identical illumination are generally preferred. However, it may be desirable to provide sequences of two or more different pulses to assist in the elimination of false targets, such as reflections from spectacles. The wavelengths may be varied between successive pulses and/or the intensity of illumination may be varied.

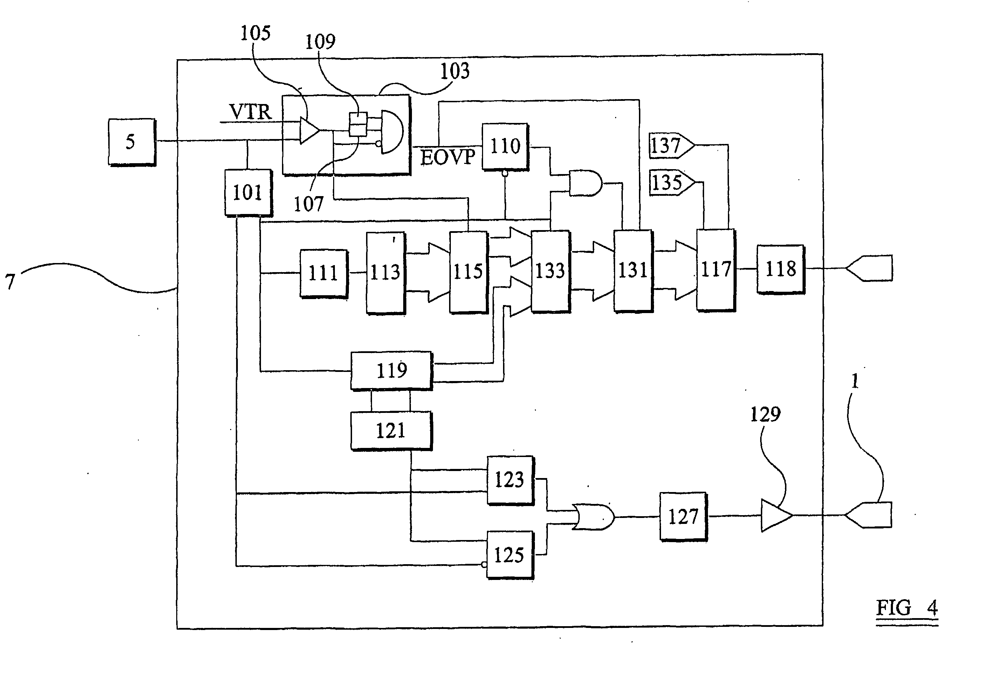

The embodiment of Figure 3 is further explained by reference to Figure 4.

The processor 7 comprises a number of components including a conventional synchronisation pulse stripper chip 101 to extract timing information relating to the line scan and fields and a pulse validator 103 including a comparator 105 which is set to select video signals having an amplitude above a predetermined threshold V_{TR}. The threshold is variable, but is generally set at about 10 to 100 percent of the saturation value, preferably about 50 percent. In practice this the predetermined threshold is about 350 millivolts. The processor 7 further comprises timer circuitry 107 which is set to reject video signals shorter than a predetermined lower threshold. The lower threshold is variable, but is generally set at about 0.5 percent of the field of view of the imaging device (for a 600 pixel device, about 3 to 4 pixels). The processor further comprises timer circuitry 109 which is set to reject video signals longer than a predetermined upper threshold. The upper threshold is variable, but is generally set at about 5 percent of the field of view of the imaging device (for a 600 pixel device, about 25 to 30 pixels). The processor 7 further includes an oscillator 111, for example operating at 7.5 MHz, to provide a horizontal dot clock, synchronised with the start of a line, to provide at 113 a horizontal (x co-ordinate) position count and circuitry 115 for providing a position count for the centre-point of the video signals satisfying the thresholds. The processor 7 further includes a microprocessor 117 for processing the centre-points calculated for the features on that line (line scan data). If centre-points appear on successive lines with substantially the same horizontal co-ordinates they are assumed to arise from a single object that is the target to be tracked. The microprocessor 117 is set to reject centre-points appearing on a number of lines less than a predetermined lower threshold. The lower threshold is variable, but is generally set at about 4 lines. The microprocessor 117 is additionally set to reject centre-points appearing on a number of lines more than a predetermined upper threshold. The upper threshold is variable, but is generally set at about 25 lines. The microprocessor 117 calculates the vertical average value of accepted centre-points (y co-ordinate) and converts the x and y co-ordinates to RS 232 data packets which are sent to a serial port 118 for onward transmission to a serial port (not shown) of a personal computer or the like.

In overview, the specific system described above operates in the following manner.

Flash illumination is synchronised to the camera shutter so as to maximise the instantaneous intensity of the radiation source 1 relative to the radiation of the background scene. Basic timing is derived from a line counter 119, the line counter output being fed to a shutter comparator 121 for providing the basic timing pulse for each flash. The basic timing pulse is used, in conjunction with detectors 123, 125 for determining odd and even fields, respectively, to drive a pulse generator 127 to provide a 100 microsecond pulse which passes to an LED driver 129 which in turn triggers the flash of the LED radiation source 1 in synchronism with the shutter.

As explained briefly above, acquisition circuitry compares the composite video input level (it should be noted other forms of input signal could be used, such as RGB) with a predetermined threshold V_{TR} and provides an output when the amplitude of the signal exceeds the threshold for a specific temporal duration. A conventional synchronisation pulse extractor 101 is used to provide vertical synchronisation, odd/even field and line synchronisation information. Vertical synchronisation is used to clear counter 119 which is subsequently incremented by the line synchronisation signal, the line counter providing vertical (y co-ordinate) position information. Voltage-controlled oscillator 111 is used to generate a horizontal dot clock at a frequency of about 7.5 MHz giving about 390 clocks during each active line period. The dot clock is resynchronised at the start of each line and is used to increment a horizontal position counter 113. When the input signal exceeds the voltage threshold V_{TR}, the pulse validator 103 issues a pulse and the current horizontal position is clocked into signal width counter 115 which is then incremented at half the dot clock rate until the input signal no longer exceeds the voltage threshold V_{TR}. The resulting count provides the horizontal position (x co-ordinate) of the centre of the active signal. Two retriggerable monostables 107 and 109 are used to verify the length of the input signal above the voltage threshold ensuring that signals which are too short or too long are rejected. If the signal is valid then the pulse validator 103 issues an "end of valid pulse" (EVOP) signal which sets a pulse latch 110 and clocks the output of the width counter 115, that is the position of the centre of the signal above the voltage threshold, into a first in first out (FIFO) buffer 131 by way of a multiplexor 133. At the end of the line, if a valid signal has been found during that line then the pulse latch 110 is set and the line number is also clocked into the FIFO buffer 131 by way of multiplexor 133.

Raw data is converted to a serial data packet using the microprocessor 117, such as an 87C51 microprocessor. The FIFO buffer 131 allows asynchronous processing of data by the microprocessor, with data only being passed to the microprocessor 117 in respect of lines which contain a valid signal. As will be apparent from the explanation above, the form in which data is passed is as one or more horizontal position counts followed by the line number. The line number is detected by the presence of a line number bit flag. The microprocessor 117 then builds 'points' out of all the valid signals provided, rejecting points which contain less than a minimum number of lines and which contain more than a maximum number of lines. Finally, the microprocessor 117 identifies the target as the best of the points found within a single field, calculates the centre for that point and sends a data packet down a serial line. The data packet may contain mouse button information from inputs 135 for the left mouse button and 137 for the right mouse button, in addition to x and y co-ordinate information, the button information being read directly from input pins of the microprocessor 117.

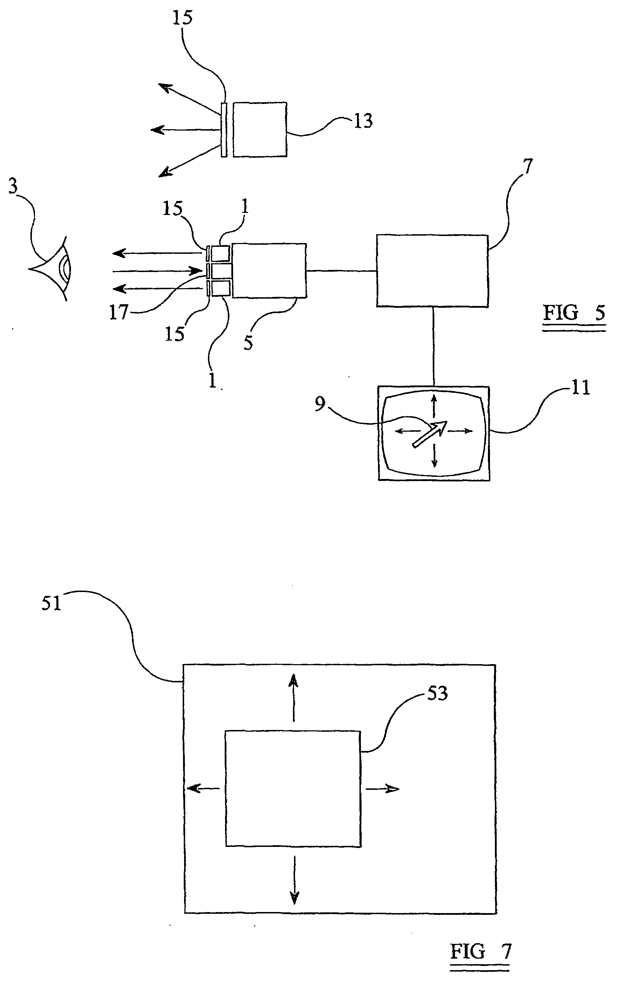

Instead of providing a separate radiation emitting device, the human eye can be employed as the optical tag using, for example, the so-called red eye phenomenon (which is a well known characteristic of flash photography). In such a case, as illustrated in Figure 5, the location of the source 1 and the imaging device should be such that the cone of retro-reflected light that is characteristic of the retro-reflector used results in a significant fraction of the reflected light being captured by the camera lens. By employing the red eye phenomenon, one or more of the eyes of a user of the apparatus shows up as a region which can be readily distinguished from the background scene as will be described hereinafter.

The imaging device 5 comprises, for example, a digital colour camera which provides a pixel-scanned output signal. In one embodiment the conventional infra-red blocking filter is removed, and additionally a visible blocking, infra-red transmissive filter may be provided, and the source emits radiation in the near infra-red region.

A disadvantage of the red eye phenomenon is that the contrast is relatively low between the red eye and the background scene. For example, reflections under near infra-red radiation also arise as a result of reflection from the user's face and from clothes, especially white clothes, and specular reflection from spectacles, cornea and teeth in addition to retroreflection from the user's eyes.

In order to provide a mechanism for better discriminating between wanted red eye reflections (targets) and unwanted reflections, a further radiation source 13 is mounted away from the axis of the imaging device and illuminates the field of view of the imaging device with radiation of a specific wavelength or wavelength range, for example radiation in the visible red region or further into the infra red region. The further radiation source 13 is mounted sufficiently close to the radiation source 1 that substantially all areas illuminated by the source 1 are also illuminated by the source 13. If necessary, more than one further source 13 may be provided, for example mounted on opposite sides of the axis of the imaging device 5. Additionally, the further radiation source 13 is mounted sufficiently far from the axis of the imaging device 5 that there is substantially no retro-reflection of radiation from the further source 13. Thus, radiation from the further radiation source 13 is reflected from all objects within the field of view of the imaging device 5 except (because it is off-axis) from the pupil or pupils of the user (although it should be noted the "red eye" system can be used with any retro-reflective target). The result of this is that all objects within the field of view which reflect the radiation from the source 1 will also reflect the radiation from the source 13 except for the pupil or pupils of the user which will reflect only the radiation from the source 1 and therefore appear "white" as explained below.

Thus, in one embodiment, the colour camera described above, with the infra-red blocking filter removed, retains its conventional red, green and blue filters. In practice, all three filters transmit radiation above about 800 nm, while the red filter also transmits radiation in the very near infra-red region between 700 and 800 nm. Thus, radiation at about 700 to 750 nm (for example 740 nm) passes through the red filter, but not through the green or blue filters, and therefore produces a substantially red image on a conventional TV monitor, while radiation at about 800 to 1000 nm (for example 810 nm) passes through all three filters (and therefore appears white on the monitor). It should be noted alternative colorisation filters can be used. For example wavelengths of 880 nm and 940 nm would be less noticeably visually.

With the 810 nm radiation source 13 mounted away from the lens of the imaging device 5 and the 740 nm radiation source 1 mounted laterally adjacent to the lens of the imaging device (or, where several radiation emitters are provided, around the lens) such that the radiation source 1 is substantially co-located with the imaging device 5, the red eye effect indeed appears substantially pure red, while other reflections are combined with white and therefore appear a rose colour. That is, the 740 nm radiation source is located such that a substantial fraction of the cone of radiation returned by the retroreflector passes through the lens of the imaging device 5 and can contribute to the image. Unfortunately, in practice if the red-eye intensity is too high, the colour information can be lost and the red-eye target can appear white and become lost. Alternatively, if the red-eye intensity is too low or the pupil is too small, the signal corresponding to the red-eye effect can be undetectable.

Alternatively, with the 740 nm radiation source 13 mounted away from the lens of the imaging device 5 and the 810 nm radiation source 1 mounted to emit radiation from one or more points substantially co-located with the imaging device, the red eye effect appears white, while other reflections are combined with red and again appear a rose colour.

It has been established that the eye is a retroreflector which does not preserve the polarisation of incident light. The retina acts as an inefficient mirror which reflects by diffuse scattering rather than by specular reflection and therefore scrambles polarisation.

Polarised light can therefore be eliminated from specular reflections, such as from spectacle frames and lenses, by inserting a first polarising filter 15 in front of each of the radiation sources 1 and 13 and a second polarising filter 17, oriented to block polarised radiation from the first polarising filter (in this case oriented substantially at right angles to the polarising axis of the first polarising filter), in front of the imaging device 5. In this way, specular reflections can be removed by the second polarising filter 17, while the diffuse reflections from the eye pass through the filter and are detected by the imaging device 5 as a target.

The signal output from the digital imaging device 5 can therefore be analysed pixel-by-pixel, each pixel being classified according to the intensity of two or more of the colour components comprising the pixel.

In an embodiment, in which on-axis radiation of 810 nm results in a white image from the pupils and face and off-axis radiation of 740 nm results in an additional red image of the face while the pupils remain black, the image may be considered as the sum of two input signals: (signal 1) white (eye or face) where the intensity of the red and green components is similar, and (signal 2) red (face) where the intensity is substantially pure red, the green component being substantially zero. The sum of the two signals formed within the imaging device (camera) is therefore white (where R=G in the region of the eyes) and pink (where R=2G over the remainder of the face). Thus, in this case the signal is analysed for the green intensity (Igr) and the red intensity (Ird).

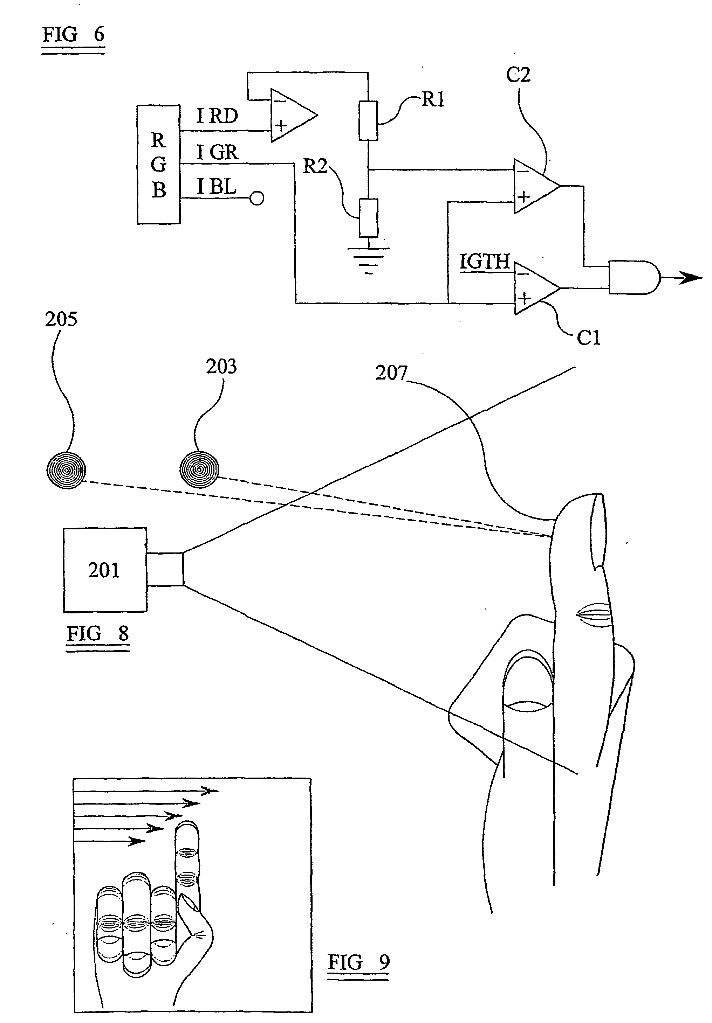

The input signal is filtered to reject all regions where Igr is less than a minimum threshold (Igth): this effectively removes all dark regions from consideration and significantly simplifies processing. This is achieved by comparator C1 shown in Figure 6 in which the circuit shown is effectively a replacement for the comparator 105 shown in Figure 4. The input signal is also tested for the ratio of the green and red intensities to filter out areas where the red intensity prevails. This is effected by calculating the ratio Igr/Ird and filtering out areas where the result exceeds a threshold (Icolour). This is achieved by comparator C2 shown in Figure 6. Typical values for the thresholds are about 0.3 for Igth and about 1.1 for Icolour. For a conventional video signal, Igth=0.3 is equivalent to a signal level of 0.21 volts and for Icolour=1.1 then R2=10xR1. The outputs of the two comparators C1 and C2 are ANDed to produce a logic level input signal which is only high in the white areas defined above. The filtering operation(s) are carried out in real time before passing the filtered input to the microprocessor 117.

It should be noted the blue component of the image signal can be used in place of the green component.

Thus, signal processing is relatively simple and undemanding, permitting relatively inexpensive components to be employed operating in real time, and location of the user's eye is straightforward without the need for conventional image processing software and hardware. Moreover, processing is effected on a single frame output from the imaging device 5 and there is no need to compare successive frames with the attendant storage and processing costs and motion-induced difficulties.

The input signal can additionally be used to determine perclos. The signal provides sufficient information to protect against the potential effects of spurious reflections, for example by using the separation and likely range of tilt of the eyes. In addition, it may be necessary to determine the shape of the reflection to determine eye-lid droop where the eye-lid obscures to top of an otherwise circular pupil.

As a further alternative, two images can be formed either sequentially or with two imaging devices, one image using substantially co-located radiation thereby generating the red eye phenomenon and the other using off-axis radiation of substantially the same wavelength range but not generating the red eye phenomenon. The two images can be processed in the manner described above, either pixel-by-pixel or by other means, to identify the required pixels. The processed images are then used to determine the x and y co-ordinates of the target.

The red eye phenomenon can additionally be used as a "blink or wink" switch or as a tilt (such as a head tilt) switch thus allowing the transmission of information other than simple x and y co-ordinate information.

Figure 7 illustrates an alternative or additional implementation of the optical monitoring apparatus to display part only of a larger image.

The larger image 51 may comprise, for example, a scene, a spreadsheet or a form that cannot be displayed as a whole on a screen 53. It should be noted the larger image is shown only for the purposes of illustration and may, for example, be stored in memory and/or may be calculated or derived as required.

Thus, if a target, or wanted reflection, is moved to the left the part of the image displayed is panned to the right to move that part of the image at the left of the display towards the centre. If the target, or wanted reflection, is moved to the right the part of the image displayed is panned to the left to move that part of the image at the right of the display towards the centre. If the target, or wanted reflection, is moved upwardly the part of the image at the top of the display is moved, or tilted, downwardly. If the target, or wanted reflection, is moved downwardly the part of the image at the bottom of the display is moved, or tilted, upwardly.

Movement of the target, or wanted reflection, can be effected alone or in combination with a cursor or the like. For example, the image can be panned or tilted when the cursor or the like is moved to an appropriate edge of the display.

The target, or wanted reflection, is moved by the user of the apparatus moving his or her head or eyes to look upwardly or downwardly and/or left or right.

The image to be displayed can be controlled in a further way which can optionally be superimposed upon the controls described hereinabove.

That is, the image to be displayed can be zoomed in or out depending on the distance of the radiation emitting device (whether an optical tag or an eye). As the radiation emitting device is moved towards the display the image displayed is progressively increased in scale (magnified), while as the radiation emitting device is moved away from the display the image displayed is progressively reduced in scale.

Movement of the radiation emitting device towards and away from the imaging device (which may be mounted on or adjacent to the display) can be determined in a number of ways.

For example, the relative size of the target generated by the radiation emitting device will change, becoming smaller as the radiation emitting device is moved away from the imaging device and growing as the radiation emitting device is moved towards the imaging device. Thus, it is possible to determine the relative size of the target to determine in turn the distance from the imaging device and consequently to determine whether any zoom function is required.

Alternatively, where more than one target is provided (such as a pair of eyes) the relative spacing of the targets generated by the radiation emitting device will change, becoming more closely spaced as the radiation emitting device is moved away from the imaging device and becoming more widely spaced as the radiation emitting device is moved towards the imaging device. Thus, it is possible to determine the relative spacing of the targets to determine in turn the distance from the imaging device and consequently to determine whether any zoom function is required.

Figure 8 shows an embodiment of the monitoring apparatus according to the present invention for identifying the location of an optical tag (target) in the form of a pointing device, such as a finger, in a 3-dimensional volume. The apparatus comprises an imaging device 201, such as a digital colour camera, set up to operate as described above in relation to Figure 5. The camera may have a 2.3 mm lens giving a field of view of about 90 degrees vertically and 110 degrees horizontally with a 12.5 mm CCD camera. A first radiation source 203 is located beside the imaging device and a second radiation source 205 is located in a plane behind the first source, also at a position beside the imaging device. The two radiation sources are point sources and emit radiation of different wavelengths, such as 740 nm for the first radiation source 203 and 810 nm for the second radiation source 205. The second radiation source may be positioned, for example, about 20 mm behind the first radiation source. It should be noted the radiation sources may either be physically positioned as shown in Figure 8 or may employ well known imaging means to create a point source at one or more suitable locations, for example at a location in front of the imaging device.

In the illustrated embodiment, such an imaging device is able to detect a target 207, such as a finger tip, at a range of about 30 mm to 90 mm. At 30 mm the field of view of the camera is about 80 mm and this defines the maximum lateral extent of the region to be monitored. Thus the target can be monitored within a volume centred on a point about 60 mm from the lens and having a movement towards or away from the lens of about 30 mm and a movement in a plane perpendicular to the optical axis of the lens of about 40 mm from the axis. It should be noted alternative ranges can be employed. Thus, a relatively small and compact sensor can be employed to detect and utilise a target, such as a human finger, in a substantial region of space. In practice the image signal is processed from the top of the field of view and the target selected is the first target to meet the selection criteria.

The intensity of radiation from each of the point sources falls off in dependence upon the the square of distance. Therefore the intensity of radiation reflected by the target will be different for each of the first and second radiation sources 203 and 205.

In the case where the second source is 20 mm behind the first source and the target is 30 mm from the lens of the imaging device, the distance travelled by radiation from the first source to the target is 30 mm, while the distance travelled by radiation from the second source is 50 mm. Therefore the ratio of intensities of the two different wavelengths is (5/3)²:1, i.e., 2.78:1. Where the target is 90 mm from the lens of the imaging device, the distance travelled by radiation from the first source to the target is 90 mm, while the distance travelled by radiation from the second source is 110 mm. Therefore the ratio of intensities of the two different wavelengths is (11/9)²:1, i.e., 1.49:1. These ratios demonstrate that there are sufficient differences in intensity of radiation received from the two sources in order to be able to determine distance of the target from the imaging device.

Figure 9 illustrates the target as viewed from the imaging device. It will be apparent the location of the target in two-dimensional space can be determined as explained above in relation to Figure 1.

Thus, the angular position of the target can be determined as explained above in relation to Figure 1, while the distance of the target from the imaging device can be determined by the ratio of intensities of the radiation originating from the first and second radiation sources, which ratio can be calculated according to the configuration of the apparatus. The angular position of the target and the distance from the imaging device can be used to determine the co-ordinates of the target in space. Movement of the target can be converted into, for example, movement of a cursor on a computer screen. In this case, the distance of the target from the imaging device can be used such that a predetermined movement of the target in a plane parallel to the imaging device results in a predetermined movement of the cursor irrespective of the distance of the target from the imaging device.

## Claims

1. A method for monitoring at least one target (3, 207), comprising the steps of providing a signal indicative of an image received by an image sensor (5, 201), and processing the image signal to determine an amplitude of the image signal and to identify any part or parts of the signal having an amplitude greater than a predetermined threshold (V_{TR}), and to identify location coordinates of the part or parts of the signal having an amplitude greater than the predetermined threshold, **characterised in that** the image signal is processed to determine the duration for which any part or parts of the image signal has an amplitude greater than the predetermined threshold (V_{TR}) and to select only the part or parts having a linear extent greater than a first predetermined value and/or less than a second predetermined value, the second predetermined value being longer than the first predetermined value.

2. A method according to claim 1, **characterised in that** the first predetermined value is about 0.5 percent of the field of view of the imaging device.

3. A method according to claim 1 or 2, **characterised in that** the second predetermined value is about 5 percent of the field of view of the imaging device.

4. A method according to any preceding claim, **characterised by** processing the signal to determine the distance of the target (3, 207) from the image sensor (5, 201) on the basis of the linear extent of the part or parts of the image signal having an amplitude greater than the predetermined threshold (V_{TR}).

5. A method according to any preceding claim, **characterised in that** two sources (1, 13, 203, 205) of electromagnetic radiation are provided, the sources having first and second wavelengths, the second wavelength being different to the first wavelength.

6. A method according to claim 5, **characterised in that** one of the first and second radiation sources (1) is substantially co-located with the image sensor (5) and the other of the first and second radiation sources (13) is offset laterally relative to the image sensor.

7. A method according to claim 5 or 6, **characterised in that** a first polarising filter (15) is positioned in front of each of the first and second radiation sources (1, 13) and a second polarising filter (17), oriented to block polarised radiation from the first polarising filter, is positioned in front of the image sensor (5).

8. A method according to any one of claims 5 to 7, **characterised by** the steps of determining the amplitude of first and second spectral regions of the image signal, determining the ratio of the amplitudes of the first and second spectral regions, and selecting only the part or parts of the image signal having a ratio of at least, or not more than, a predetermined value, for example less than about 1.1.

9. A method according to claim 5, **characterised in that** the first and second radiation sources (203, 205) are arranged in first and second planes parallel to the image sensor, the first and second planes being spaced in the direction of an optical axis of the image sensor (201).

10. A method according to claim 9, **characterised in that** the first and second radiation sources (203, 205) are substantially co-located with the image sensor (201).

11. A method according to claim 9 or 10, **characterised by** determining the coordinates of the target (207) in space on the basis of the angular position of the target and the distance thereof from the image sensor (201).

12. An apparatus for monitoring at least one target (3, 207), comprising an image sensor (5, 201) for producing a signal indicative of an image received by the sensor, and means (7) for processing the image signal, the processing means including comparator means (105) for determining an amplitude of the image signal and for identifying any part or parts of the signal having an amplitude greater than a predetermined threshold (V_{TR}), and including means (113, 119) for identifying location coordinates of the part or parts of the signal having an amplitude greater than the predetermined threshold, **characterised in that** the processing means (7) includes means (107, 109) for determining the duration for which any part or parts of the image signal has an amplitude greater than the predetermined threshold (V_{TR}) and for selecting only the part or parts having a linear extent greater than a first predetermined value and/or less than a second predetermined value, the second predetermined value being longer than the first predetermined value.

13. An apparatus as claimed in claim 12, **characterised in that** the first predetermined value is about 0.5 percent of the field of view of the imaging device.

14. An apparatus as claimed in claim 12 or 13,
**characterised in that** the second predetermined value is about 5 percent of the field of view of the imaging device.

15. An apparatus as claimed in claim 12, 13 or 14, **characterised in that** the processing means (7) includes means for determining the distance of the target from the image sensor on the basis of the linear extent of the part or parts of the image signal having an amplitude greater than the predetermined threshold (V_{TR}).

16. An apparatus as claimed in any one of claims 12 to 15, **characterised in that** two sources (1, 13, 203, 205) of electromagnetic radiation are provided, the sources having first and second wavelengths, the second wavelength being different to the first wavelength.

17. An apparatus as claimed in claim 16, **characterised in that** one of the first and second radiation sources (1) is substantially co-located with the image sensor (5) and the other of the first and second radiation sources (13) is offset laterally relative to the image sensor.

18. An apparatus as claimed in claim 16 or 17, **characterised in that** a first polarising filter (15) is positioned in front of each of the first and second radiation sources (1, 13) and a second polarising filter (17), oriented to block polarised radiation from the first polarising filter, is positioned in front of the image sensor (5).

19. An apparatus as claimed in any one of claims 16 to 18, **characterised in that** the processing means (7) includes means for determining the amplitude of first and second spectral regions of the image signal, means for determining the ratio of the amplitudes of the first and second spectral regions, and means for selecting only the part or parts of the image signal having a ratio of at least, or not more than, a predetermined value, for example less than about 1.1.

20. An apparatus as claimed in claim 16, **characterised in that** the first and second radiation sources (203, 205) are arranged in first and second planes parallel to the image sensor, the first and second planes being spaced in the direction of an optical axis of the image sensor (201).

21. An apparatus as claimed in claim 20, **characterised in that** the first and second radiation sources (203, 205) are substantially co-located with the image sensor (201).

22. An apparatus as claimed in claim 20 or 21, **characterised in that** the processing means includes means for determining the coordinates of the target (207) in space on the basis of the angular position of the target and the distance thereof from the image sensor (201).

## Patentansprüche

1. Verfahren zum Überwachen von wenigstens einem Ziel (3, 207), das die folgenden Schritte umfasst: Erzeugen eines Signals, das ein von einem Bildsensor (5, 201) empfangenes Bild anzeigt, und Verarbeiten des Bildsignals, um eine Amplitude des Bildsignals zu ermitteln und um ein oder mehrere Teile des Signals mit einer Amplitude zu identifizieren, die größer ist als ein vorbestimmter Schwellenwert (V_{TR}), und um Ortskoordinaten des Teils oder der Teile des Signals mit einer Amplitude zu identifizieren, die größer ist als der vorbestimmte Schwellenwert, **dadurch gekennzeichnet, dass** das Bildsignal verarbeitet wird, um die Dauer zu ermitteln, während der (ein) Teil(e) des Bildsignals eine Amplitude hat/haben, die größer ist als der vorbestimmte Schwellenwert (V_{TR}), und um nur den Teil oder die Teile auszuwählen, der/die ein lineares Ausmaß hat/haben, das größer als ein erster vorbestimmter Wert und/oder kleiner als ein zweiter vorbestimmter Wert ist, wobei der zweite vorbestimmte Wert länger ist als der erste vorbestimmte Wert.

2. Verfahren nach Anspruch 1, **dadurch gekennzeichnet, dass** der erste vorbestimmte Wert etwa 0,5 % des Sichtfeldes des Abbildungsgerätes beträgt.

3. Verfahren nach Anspruch 1 oder 2, **dadurch gekennzeichnet, dass** der zweite vorbestimmte Wert etwa 5 % des Sichtfeldes des Abbildungsgerätes beträgt.

4. Verfahren nach einem der vorherigen Ansprüche, **gekennzeichnet durch** Verarbeiten des Signals, um den Abstand des Ziels (3, 207) vom Bildsensor (5, 201) auf der Basis des linearen Ausmaßes des Teils oder der Teile des Bildsignals zu ermitteln, der/die eine Amplitude hat/haben, die größer ist als der vorbestimmte Schwellenwert (V_{TR}).

5. Verfahren nach einem der vorherigen Ansprüche, **dadurch gekennzeichnet, dass** zwei Quellen (1, 13, 203, 205) von elektromagnetischer Strahlung vorgesehen sind, wobei die Quellen eine erste und eine zweite Wellenlänge haben, wobei die zweite Wellenlänge sich von der ersten Wellenlänge unterscheidet.

6. Verfahren nach Anspruch 5, **dadurch gekennzeichnet, dass** die erste oder die zweite Strahlungsquelle (1) sich im Wesentlichen am selben Ort befindet wie der Bildsensor (5) und die andere aus der ersten und der zweiten Strahlungsquelle (13) relativ zum Bildsensor lateral versetzt ist.

7. Verfahren nach Anspruch 5 oder 6, **dadurch gekennzeichnet, dass** ein erster Polarisationsfilter (15) jeweils vor der ersten und der zweiten Strahlungsquelle (1, 13) und ein zweiter Polarisationsfilter (17), der so ausgerichtet ist, dass er polarisierte Strahlung vom ersten Polarisationsfilter sperrt, vor dem Bildsensor (5) positioniert wird.

8. Verfahren nach einem der Ansprüche 5 bis 7, **gekennzeichnet durch** die folgenden Schritte: Ermitteln der Amplitude des ersten und des zweiten Spektralbereichs des Bildsignals, Ermitteln des Verhältnisses zwischen den Amplituden des ersten und des zweiten Spektralbereichs und Auswählen von nur dem Teil oder den Teilen des Bildsignals mit einem Verhältnis von wenigstens, oder nicht mehr als, einem vorbestimmten Wert, z.B. von weniger als etwa 1,1.

9. Verfahren nach Anspruch 5, **dadurch gekennzeichnet, dass** die erste und die zweite Strahlungsquelle (203, 205) in einer ersten und einer zweiten Ebene parallel zum Bildsensor angeordnet sind, wobei die erste und die zweite Ebene in der Richtung einer optischen Achse des Bildsensors (201) beabstandet sind.

10. Verfahren nach Anspruch 9, **dadurch gekennzeichnet, dass** die erste und die zweite Strahlungsquelle (203, 205) sich im Wesentlichen am selben Ort befinden wie der Bildsensor (201).

11. Verfahren nach Anspruch 9 oder 10, **gekennzeichnet durch** das Ermitteln der Koordinaten des Ziels (207) im Raum auf der Basis der Winkelposition des Ziels und dessen Abstands von dem Bildsensor (201).

12. Vorrichtung zum Überwachen von wenigstens einem Ziel (3, 207), umfassend einen Bildsensor (5, 201) zum Erzeugen eines Signals, das ein von dem Sensor empfangenes Bild anzeigt, und Mittel (7) zum Verarbeiten des Bildsignals, wobei das Verarbeitungsmittel Komparatormittel (105) zum Ermitteln einer Amplitude des Bildsignals und zum Identifizieren eines oder mehrerer Teile des Signals mit einer Amplitude beinhaltet, die größer ist als ein vorbestimmter Schwellenwert (V_{TR}), und mit Mitteln (113, 119) zum Identifizieren von Ortskoordinaten des Teils bzw. der Teile des Signals mit einer Amplitude, die größer ist als der vorbestimmte Schwellenwert, **dadurch gekennzeichnet, dass** das Verarbeitungsmittel (7) Mittel (107, 109) zum Ermitteln der Dauer beinhaltet, für die (ein) Teil(e) des Bildsignals eine Amplitude hat/haben, die größer ist als der vorbestimmte Schwellenwert (V_{TR}), und um nur das/die Teil(e) mit einem linearen Ausmaß auszuwählen, das größer als ein erster vorbestimmter Wert und/oder kleiner als ein zweiter vorbestimmter Wert ist, wobei der zweite vorbestimmte Wert länger ist als der erste vorbestimmte Wert.

13. Vorrichtung nach Anspruch 12, **dadurch gekennzeichnet, dass** der erste vorbestimmte Wert etwa 0,5 % des Sichtfeldes des Abbildungsgerätes beträgt.

14. Vorrichtung nach Anspruch 12 oder 13, **dadurch gekennzeichnet, dass** der zweite vorbestimmte Wert etwa 5 % des Sichtfeldes des Abbildungsgerätes beträgt.

15. Vorrichtung nach Anspruch 12, 13 oder 14, **dadurch gekennzeichnet, dass** das Verarbeitungsgerät (7) Mittel zum Ermitteln des Abstands des Ziels vom Bildsensor auf der Basis des linearen Ausmaßes des Teils oder der Teile des Bildsignals beinhaltet, das/die eine Amplitude hat/haben, die größer ist als der vorbestimmte Schwellenwert (V_{TR}).

16. Vorrichtung nach einem der Ansprüche 12 bis 15, **dadurch gekennzeichnet, dass** zwei Quellen (1, 13, 203, 205) von elektromagnetischer Strahlung vorgesehen sind, wobei die Quellen eine erste und eine zweite Wellenlänge haben, wobei die zweite Wellenlänge sich von der ersten Wellenlänge unterscheidet.

17. Vorrichtung nach Anspruch 16, **dadurch gekennzeichnet, dass** sich die erste oder die zweite Strahlungsquelle (1) im Wesentlichen am selben Ort befindet wie der Bildsensor (5) und die andere aus der ersten und der zweiten Strahlungsquelle (13) relativ zum Bildsensor lateral versetzt ist.

18. Vorrichtung nach Anspruch 16 oder 17, **dadurch gekennzeichnet, dass** ein erster Polarisationsfilter (15) jeweils vor der ersten und der zweiten Strahlungsquelle (1, 13) und ein zweiter Polarisationsfilter (17), der so ausgerichtet ist, dass er polarisierte Strahlung vom ersten Polarisationsfilter sperrt, vor dem Bildsensor (5) positioniert ist.

19. Vorrichtung nach einem der Ansprüche 16 bis 18, **dadurch gekennzeichnet, dass** das Verarbeitungsmittel (7) Mittel zum Ermitteln der Amplitude des ersten und des zweiten Spektralbereichs des Bildsignals, Mittel zum Ermitteln des Verhältnisses zwischen den Amplituden des ersten und des zweiten Spektralbereichs und Mittel zum Auswählen nur des Teils oder der Teile des Bildsignals beinhaltet, das/die ein Verhältnis von wenigstens, oder nicht mehr als, einem vorbestimmten Wert hat/haben, z.B. von weniger als etwa 1,1.

20. Vorrichtung nach Anspruch 16, **dadurch gekennzeichnet, dass** die erste und die zweite Strahlungsquelle (203, 205) in einer ersten und einer zweiten Ebene parallel zum Bildsensor angeordnet sind, wobei die erste und die zweite Ebene in der Richtung einer optischen Achse des Bildsensors (201) beabstandet sind.

21. Vorrichtung nach Anspruch 20, **dadurch gekennzeichnet, dass** sich die erste und die zweite Strahlungsquelle (203, 205) im Wesentlichen am selben Ort befinden wie der Bildsensor (201).

22. Vorrichtung nach Anspruch 20 oder 21, **dadurch gekennzeichnet, dass** das Verarbeitungsmittel Mittel zum Ermitteln der Koordinaten des Ziels (207) im Raum auf der Basis der Winkelposition des Ziels und dessen Abstands vom Bildsensor (201) beinhalten.

## Revendications

1. Un procédé pour surveiller au moins une cible (3, 207), comprenant les étapes qui consistent à fournir un signal indicatif d'une image reçue par un capteur d'images (5, 201), et à traiter le signal d'image pour déterminer une amplitude du signal d'image et pour identifier toute partie ou toutes parties du signal dont l'amplitude excède un seuil prédéterminé (V_{TR}) et pour identifier les coordonnées de position de la partie ou des parties du signal dont l'amplitude excède le seuil prédéterminé, **caractérisé en ce que** le signal d'image est traité pour déterminer la durée pendant laquelle une partie ou des parties quelconque(s) de signal d'image a (ont) une amplitude plus grande que le seuil prédéterminé V_{TR}) et pour ne sélectionner que la ou les partie(s) ayant une étendue linéaire supérieure à une première valeur prédéterminée, et/ou inférieure à une deuxième valeur prédéterminée la deuxième valeur prédéterminée étant plus longue que la première valeur prédéterminée.

2. Un procédé selon la revendication 1, **caractérisé en ce que** la première valeur prédéterminée égale 0,5 pour cent environ du champ visualisé par le dispositif d'imagerie.

3. Un procédé selon la revendication 1 ou 2, **caractérisé en ce que** la deuxième valeur prédéterminée égale 5 pour cent environ du champ visualisé par le dispositif d'imagerie.

4. Un procédé selon l'une quelconque des revendications précédentes, **caractérisé par** le traitement du signal pour déterminer la distance entre la cible (3, 207) et le capteur d'images (5, 201) sur la base de l'étendue linéaire de la partie ou des parties du signal d'image ayant une amplitude supérieure au seuil prédéterminé(V_{TR}).

5. Un procédé selon l'une quelconque des revendications précédentes, **caractérisé en ce que** deux sources (1, 13, 203, 205) de rayonnement électromagnétique sont prévues, les sources ayant une première et une deuxième longueurs d'ondes, la deuxième longueur d'onde différant de la première longueur d'onde.

6. Un procédé selon la revendication 5, **caractérisé en ce que** l'une des première et deuxième sources de rayonnement (1) est installée sensiblement au même endroit que le capteur d'images (5) et l'autre source de rayonnement (13) est décalée latéralement relativement au capteur d'images (5).

7. Un procédé selon la revendication 5 ou 6, **caractérisé en ce qu'**un premier polariseur (15) est placé devant chacune des première et deuxième sources de rayonnement (1, 13) et un deuxième polariseur (17), orienté de sorte à bloquer le rayonnement polarisé provenant du premier polariseur, est placé devant le capteur d'images (5).

8. Un procédé selon l'une quelconque des revendications 5 à 7, **caractérisé par** les étapes consistant à déterminer l'amplitude des première et deuxième régions spectrales du signal d'image, à déterminer le rapport des amplitudes des première et deuxième régions spectrales, et à ne sélectionner que la partie ou les parties du signal d'image ayant un rapport au moins égal et non supérieur à une valeur prédéterminée, moins de 1,1 environ par exemple.

9. Un procédé selon la revendication 5, **caractérisé en ce que** les première et deuxième sources de rayonnement (203, 205) sont agencées dans un premier et un deuxième plans parallèles au capteur d'images, les premier et deuxième plans étant espacés dans la direction d'un axe optique du capteur d'images (201).

10. Un procédé selon la revendication 9, **caractérisé en ce que** les première et deuxième sources de rayonnement (203, 205) sont installées sensiblement au même endroit que le capteur d'images (201).

11. Un procédé selon la revendication 9 ou 10, **caractérisé par** la détermination des coordonnées spatiales de la cible (207) sur la base de la position angulaire de la cible et de sa distance relativement au capteur d'images (201).

12. Un appareil pour surveiller au moins une cible (3, 207), comprenant un capteur d'images (5, 201) pour produire un signal indicatif d'une image reçue par le capteur, et des moyens (7) pour traiter le signal d'image, les moyens de traitement comprenant des moyens comparateurs (105) pour déterminer une amplitude du signal d'image et pour identifier une partie ou des parties quelconque(s) du signal dont l'amplitude excède un seuil prédéterminé (V_{TR}) et comprenant des moyens (113, 119) pour identifier des coordonnées de position de la partie ou des parties du signal dont l'amplitude excède le seuil prédéterminé, **caractérisé en ce que** les moyens de traitement (7) comprennent des moyens (107, 109) pour déterminer la durée pendant laquelle l'amplitude d'une partie ou de parties quelconque(s) du signal excède le seuil prédéterminé (V_{TR}) et pour ne sélectionner que la ou les parties dont l'étendue linéaire est supérieure à une première valeur prédéterminée, et/ou inférieure à une deuxième valeur prédéterminée, la deuxième valeur prédéterminée étant plus longue que la première valeur prédéterminée.

13. Un appareil selon la revendication 12, **caractérisé en ce que** la première valeur prédéterminée égale environ 0,5 pour cent du champ visualisé par le dispositif d'imagerie.

14. Un appareil selon la revendication 12 ou 13, **caractérisé en ce que** la deuxième valeur prédéterminée égale environ 5 pour cent du champ visualisé par le dispositif d'imagerie.

15. Un appareil selon la revendication 12, 13 ou 14, **caractérisé en ce que** les moyens de traitement (7) comprennent des moyens pour déterminer la distance entre la cible et le capteur d'images sur la base de l'étendue linéaire de la partie ou des parties du signal d'image dont l'amplitude excède le seuil prédéterminé (V_{TR}).

16. Un appareil selon l'une quelconque des revendications 12 à 15, **caractérisé en ce que** deux sources (1, 13, 203, 205) de rayonnement électromagnétique sont prévues, les sources ayant une première et une deuxième longueurs d'ondes, la deuxième longueur d'onde différant de la première longueur d'onde.

17. Un appareil selon la revendication 16, **caractérisé en ce que** l'une des première et deuxième sources de rayonnement (1) est installée sensiblement au même endroit que le capteur d'images (5) et l'autre source de rayonnement (13) est décalée relativement au capteur d'images (5).

18. Un appareil selon la revendication 16 ou 17, **caractérisé en ce qu'**un premier polariseur (15) est placé devant chacune des première et deuxième sources de rayonnement (1, 13) et un deuxième polariseur (17), orienté pour bloquer le rayonnement polarisé qui provient du premier polariseur, est placé devant le capteur d'images (5).

19. Un appareil selon l'une quelconque des revendications 16 à 18, **caractérisé en ce que** les moyens de traitement (7) comprennent des moyens pour déterminer l'amplitude des première et deuxième régions spectrales du signal d'image, des moyens pour déterminer le rapport des amplitudes des première et deuxième régions spectrales, et des moyens pour ne sélectionner que la partie ou les parties du signal d'image ayant un rapport au moins égal ou non supérieur à une valeur prédéterminée, moins de 1,1 environ, par exemple.

20. Un appareil selon la revendication 16, **caractérisé en ce que** la première et la deuxième sources de rayonnement (203, 205) sont agencées dans les premier et deuxième plans parallèles au capteur d'images, les premier et deuxième plans étant espacés dans la direction d'un axe optique du capteur d'images (201).

21. Un appareil selon la revendication 20, **caractérisé en ce que** les première et deuxième sources de rayonnement (203, 205) sont installées sensiblement au même endroit que le capteur d'images (201).

22. Un appareil selon la revendication 20 ou 21, **caractérisé en ce que** les moyens de traitement comprennent des moyens pour déterminer les coordonnées spatiales de la cible (207) sur la base de la position angulaire de la cible et de sa distance relativement au capteur d'images (201).
